# EUROPEAN PATENT SPECIFICATION

(11) **EP 4 313 189 B1**
(45) Date of publication and mention of the grant of the patent: **15.07.2026**
(21) Application number: 22718105.4
(22) Date of filing: 23.03.2022
(51) Int. Cl.: A61L 27/14, A61L 27/50, A61L 27/56, A61F 2/07

(54) **KINK RESISTANCE TUBULAR GRAFT IMPLANTS**
KNICKBESTÄNDIGE ROHRFÖRMIGE IMPLANTATIMPLANTATE
IMPLANTS DE GREFFE TUBULAIRE RÉSISTANTS AU PLIAGE

(30) Priority: 23.03.2021 US 202163164657 P
(43) Date of publication of application: 07.02.2024
(73) Proprietor: Xeltis AG, 8008 Zurich (CH)
(72) Inventor: GRAY, Yonatan, 75011 Paris (FR); COX, Martijn Antonius Johannes, 6021 RC Budel (NL)
(74) Representative: FRKelly
(86) International application number: PCT/EP2022/057599
(87) International publication number: WO 2022/200409

(56) References cited:
- WO-A1-2013/151778
- US-B2- 8 057 535
- US-B2- 9 290 742

## Description

### FIELD OF THE INVENTION

This invention relates to kink resistant tubular graft implants and methods of making such graft implants.

### BACKGROUND OF THE INVENTION

Conventional grafts are made from various biocompatible plastics and polymers. Such devices are known to cause irritation and unwanted biologic responses from the surrounding tissues and in the lumen. Bioabsorbable and biodegradable materials have emerged more recently as a material for medical devices. For tubular vascular grafts it is important to provide sufficient mechanical properties. At the same time, it is preferred to use biodegradable materials. Therefore, a fully bioresorbable set-up would be preferred.

In US8057535 an implantable medical device comprising a fibrous polymer body is described with a support filament wrapped around the body and an outer layer around the filament for adhering the filament to the body. The wrapped filament is configured to provide a degree of anti-kinking resistance to the device. A support filament can cause irritation and other complications after implantation and is not biodegradable in the same way as the graft material. US8057535 is teaching that the support filament will be fixed to the electrospun material of the graft by a covering composition - essentially all fibers will be attached to each other.

WO 2013/151778 discloses a multi-layered tubular composite prosthetic device comprising at least one porous layer comprising electrospun poly(tetrafluoroethylene) and at least one porous layer comprising a second electrospun polymer.

An improved vascular graft needs a uniform thickness distribution through the entire length to provide good performance and additional support structures should be avoided for uniform degradation behavior. Nevertheless, tubes should not kink when bended. Standard tubes produced by electrospinning on a mandrel with a small diameter do not provide sufficient kink-resistance.

### SUMMARY OF THE INVENTION

A tubular electrospun graft implant according to claim 1 is provided distinguishing an electrospun tubular layer which has a longitudinal axis and an outer surface. One or more polymer layers of an electrospun sheet have been rolled over the outer surface of the electrospun tubular layer and around the longitudinal axis of the electrospun tubular layer. A polymer layer electrospun is further distinguished over the rolled one or more polymer layers of the electrospun sheet.

In one embodiment, the number of layers is between 10 to 20 layers. The exact number depends on the thickness that still yields uniform results.

In yet another embodiment, the implant has between 10 to 20 layers.

In yet another embodiment, each of the two or more layers has a thickness between preferably 30 micrometers to 50 micrometers or more generally a thickness between 20 micrometers to 100 micrometers.

In still another embodiment, the layers have different fiber directions relative to each other.

Embodiments of the invention have at least the following advantages:
- The graft has a significantly higher burst pressure compared to a regular spun graft.
- The graft has crushing zones created between the multiple layers that reduce the bending stiffness and facilitate bending with superior kink resistance.
- The wall thickness is more uniform and thus performs better.
- An additional support element (e.g. metal stent, polymeric stent, polymer filaments, or the like) is not necessary and the resulting device will be fully bioabsorbable.
- The multiple layers facilitate scaling up manufacturing as large sheets can be produced and cut to size and rolled into a multiple layer graft.

### BRIEF DESCRIPTION OF THE DRAWINGS

- **FIG. 1**: shows according to an exemplary embodiment of the invention silicon tube **120** placed over rod **110.** Rod **110** is used as an electrospinning mandrel/collector. For clarification, a cross section view (not to scale) is added to show the order of rod **110** and silicon tube **120.**
- **FIG. 2**: shows according to an exemplary embodiment of the invention an electrospun Polyethylene Oxide (PEO) layer **130** electrospun over silicon tube **120.** For clarification, a cross section view (not to scale) is added to show the order of silicon tube **120** and PEO layer **130.**
- **FIG. 3**: shows according to an exemplary embodiment of the invention electrospun polymer layer **140** electrospun over the electrospun PEO layer **130.** For clarification, a cross section view (not to scale) is added to show the order of PEO layer **130** and polymer layer **140.**
- **FIG. 4**: shows according to an exemplary embodiment of the invention the smooth inner/luminal surface of the graft implant, which is important for endogenous tissue regeneration (ETR).
- **FIG. 5**: shows according to an exemplary embodiment of the invention where structure **500** is removed from rod **110.** For clarification, a cross section view (not to scale) is added to show the order of the structure with silicon tube **120,** PEO layer **130** and polymer layer **140.**
- **FIG. 6**: shows according to an exemplary embodiment of the invention structure **500** from **FIG. 5** shaped around a collector **610** in a ring-shaped structure **620 (****FIG. 6****).** The length of ring-shaped structure **620** fits the circumference of collector **610.** For clarification, a cross section view (not to scale) is added to show the order of collector **610,** ring-shaped structure **620** held in ring-shape by rigid pin **630.**
- **FIG. 7**: shows according to an exemplary embodiment of the invention a layer of a polymer **710** electrospun over collector **610.** For clarification, a cross section view (not to scale) is added to show the order of collector **610** with polymer layer **710.**
- **FIG. 8**: shows according to an exemplary embodiment of the invention and with respect to **FIG. 7****,** ring-shaped structure **620** thickened by polymer **710** as it was rolled from left to right resulting in a thicker ring-shaped structure **810.**
- **FIG. 9**: shows according to an exemplary embodiment of the invention ring-shaped structure **810** cut, removed from collector **610** and the flexible wire removed resulting in cut ring-shaped structure **900.**
- **FIG. 10**: shows according to an exemplary embodiment of the invention polymer **1010** electrospun over the outer surface of structure **900** resulting in structure **1000**

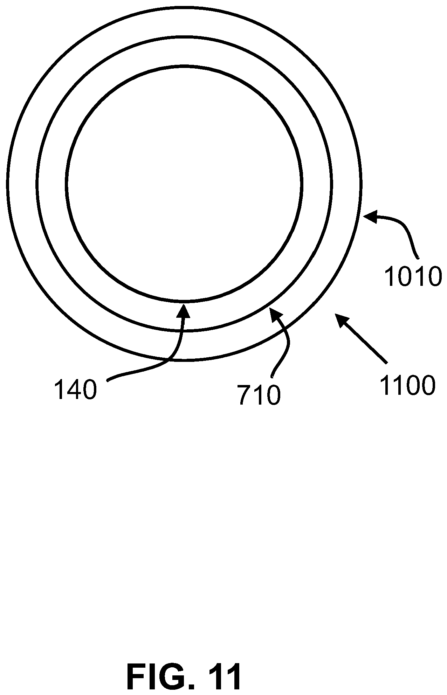

- **FIG. 11**: shows according to an exemplary embodiment of the invention structure silicon tube **120** removed from structure **1000** by dissolving Polyethylene Oxide (PEO) layer **130** resulting in structure **1100.**
- **FIG. 12**: shows according to an exemplary embodiment of the invention actual tubular graft implants manufactured by the method described herein.
- **FIG. 13**: shows according to an exemplary embodiment of the invention a prototype with a defined diameter of 4.0mm dictated by a fixed silicone mandrel. The wall thickness is ~500 µm. This prototype kinked at 1.75 cm which is still a substantial increase compared to a standard e-spun graft (without metal support) kinking before 3 cm already.
- **FIG. 14**: shows according to an exemplary embodiment of the invention an optional embodiment where the rolling is done with a sheet that is cut in an angle to the preferred fiber orientation.
- **FIG. 15**: shows fiber direction which is preferably axial which results in a preferably circumferential fiber direction following the method according to the present invention.

### DETAILED DESCRIPTION

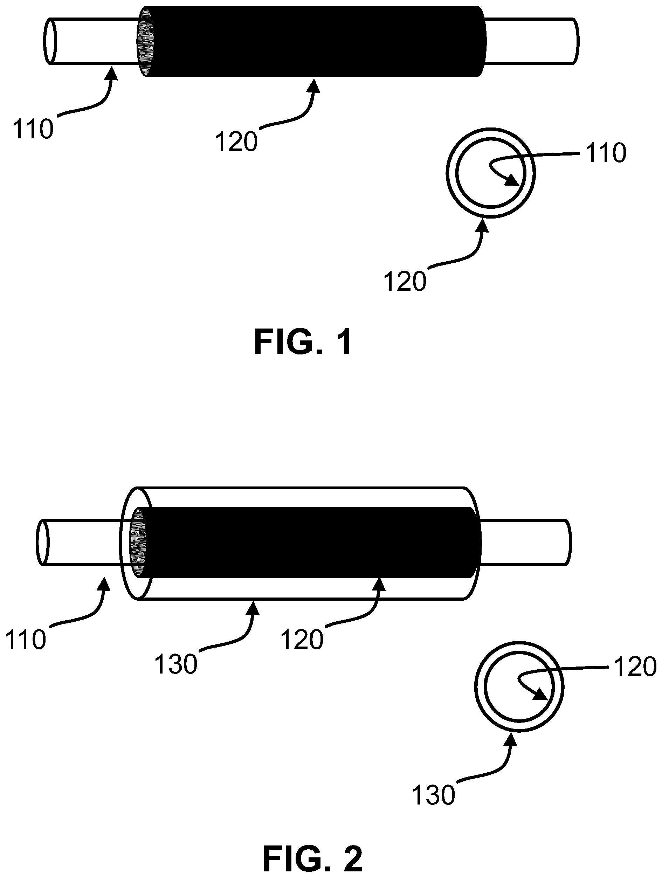

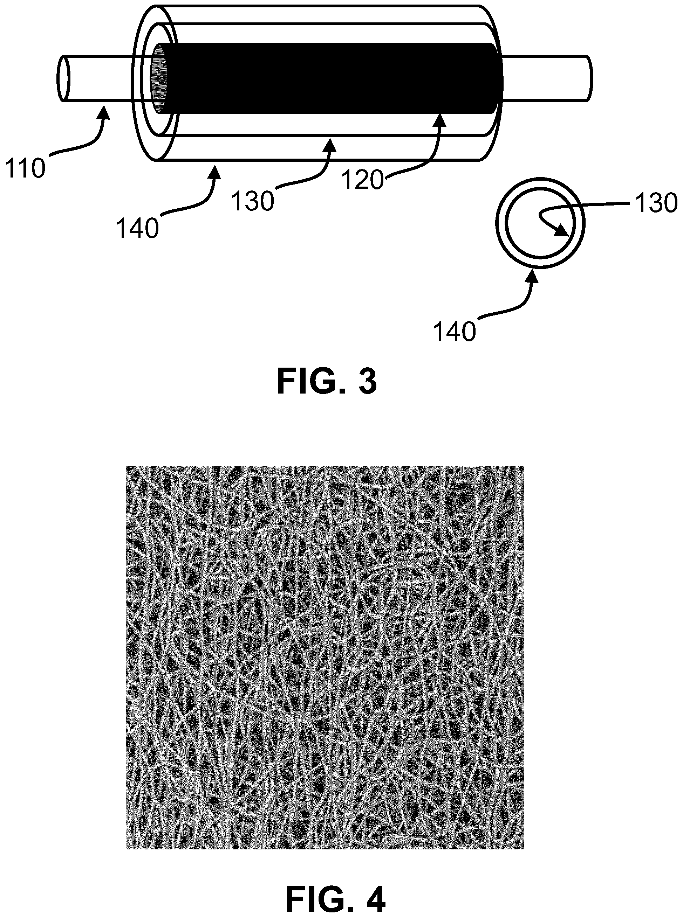

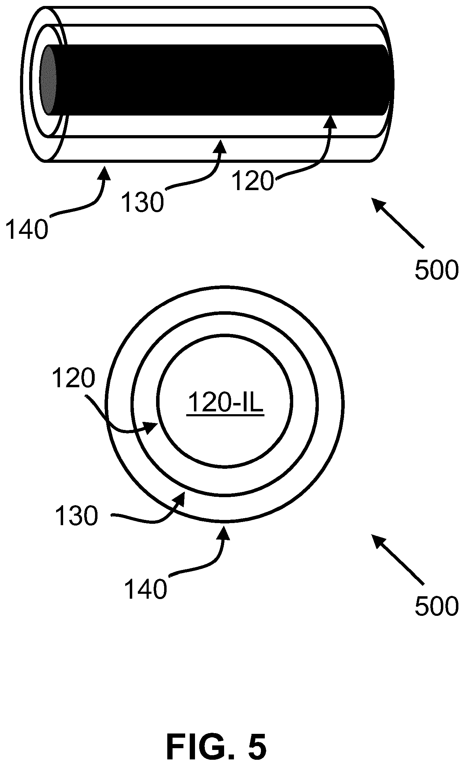

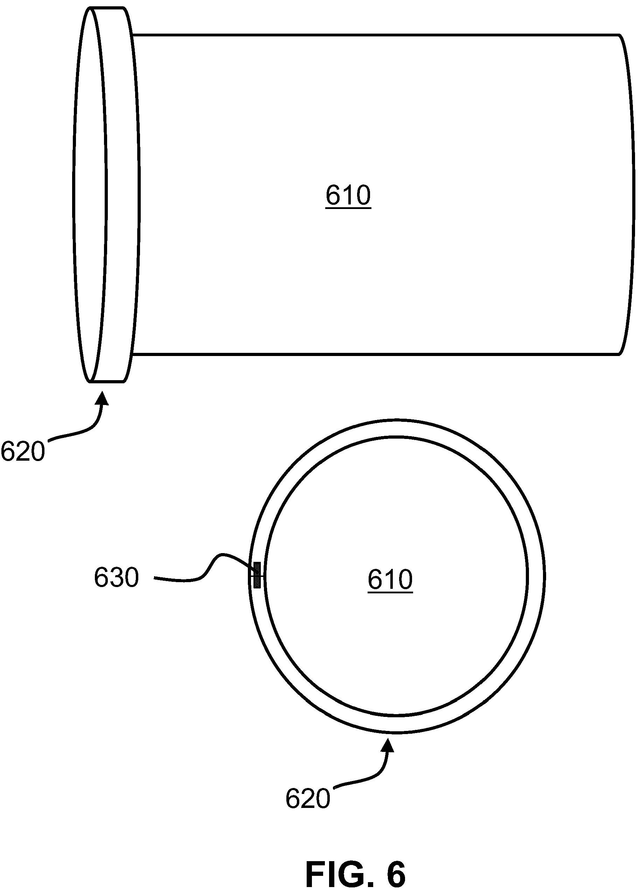

The present invention is directed to tubular graft implants made of electrospun fibers that are far more kink resistant than existing graft implants. Specifically, the method of making these tubular graft implants distinguishes the following exemplary steps:
1. In one example, a silicon tube (SI) **120** is placed on a rod **110** (e.g. a stainless-steel rod) **(****FIG. 1****).** In general, **120** could be any flexible non-metal tube and is not restricted to the exemplary silicon tube. Rod **110** is used as an electrospinning mandrel/collector. The silicon tube **120** has an outer diameter equivalent to the inner diameter of the graft implant. The length of the silicon tube is equivalent to the length of the graft implant. For example, a 40 cm long graft implant would have a silicon tube **120** of 40 cm length. Furthermore, the diameter of silicon tube **120** plus the thickness of the PEO layer (see next) will also determine the inner diameter of the graft implant.
2. In a specific example, a Polyethylene Oxide (PEO) layer **130** is electrospun over silicon tube **120 (****FIG. 2****).** PEO layer is an anti-friction water-dissolvable layer, which will later on be dissolved to remove silicon tube **120** from the tubular graft. The electrospun PEO layer is about 50 micrometers in thickness. PEO is preferred, but other materials that are water dissolvable - either electrospun or applied otherwise - could be used as a skilled artisan would readily appreciate. More generally said, this step is to facilitate removal of the silicon tube **120** from the tubular graft in a later step of the process.
3. A layer of polymer **140** is electrospun over the electrospun PEO layer **130 (****FIG. 3****).** The polymer layer **140** creates an inner/luminal surface of the graft implant. An example of the inner surface is shown in **FIG. 4****,** which is important for endogenous tissue restoration (ETR). Polymer layer **140** is intended to allow for a continuous inner lumen without the risk of delamination. A continuous lumen is important for achieving stable, laminar blood flow through the graft.
4. Structure **500** is shown as being removed from rod **110** (compare **FIG. 4** and **FIG. 5****).**
5. A rigid pin **630** will now be inserted in the lumen where rod **110** used to be and was removed from structure **500 (****FIG. 6****)** The diameter of rigid pin **630** fits the inner lumen of silicon tube **120,** indicated as **120-IL.** Purpose of pin **630** is to connect the ends of the tube to form a ring-like structure.
6. The purpose of rigid pin **630** is to shape, mount and fixate structure around a collector **610** in a ring-shape **(****FIG. 6****)** forming ring-shaped structure **620.** Alternative methods for this purpose could be considered, including but not limited to suturing or gluing. Collector **610** is for example 9 cm in diameter. The length of ring-shaped structure **620** fits the circumference of collector **610.** Differently stated, the length of the ring-shaped structure **620** and the circumference of collector **610** should fit each other and are equal to the length of the resulting graft implant.

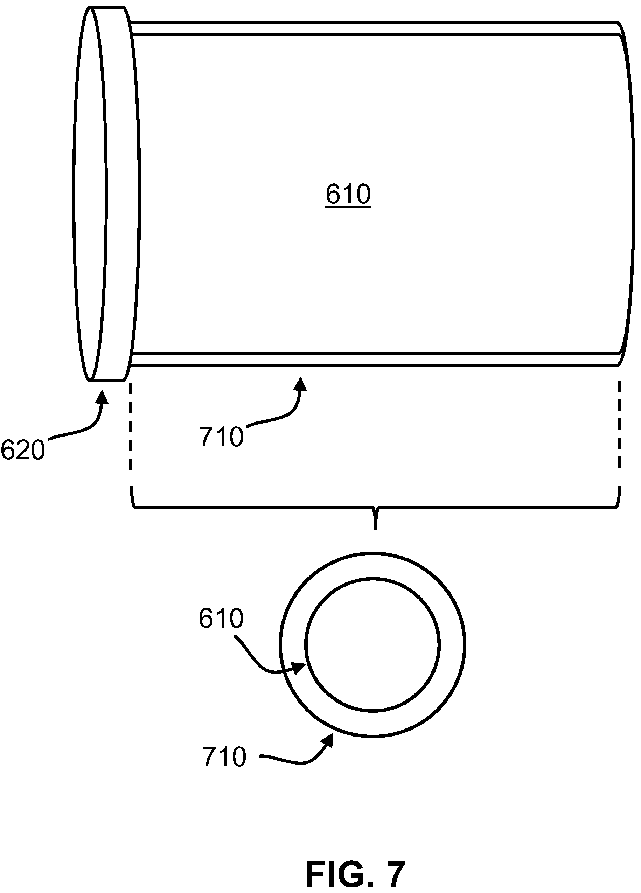

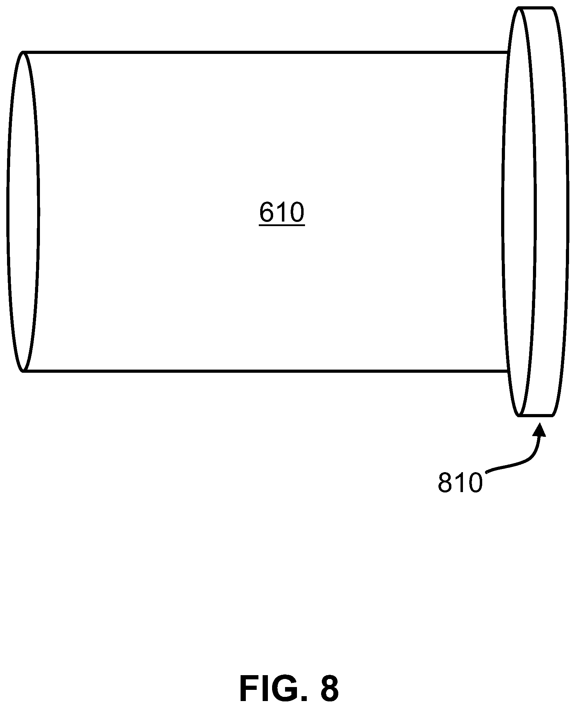

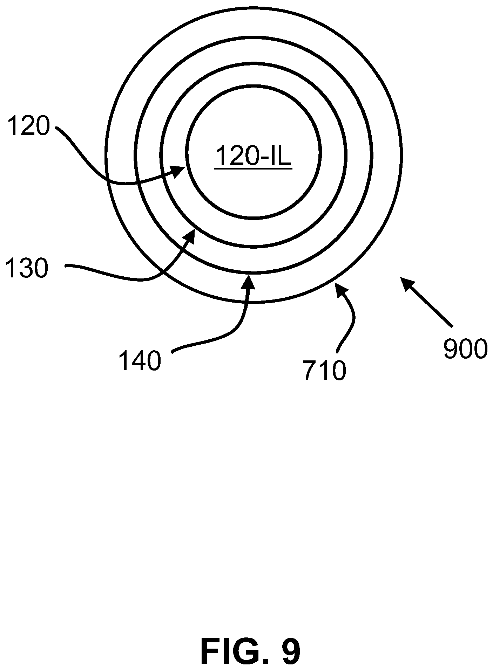

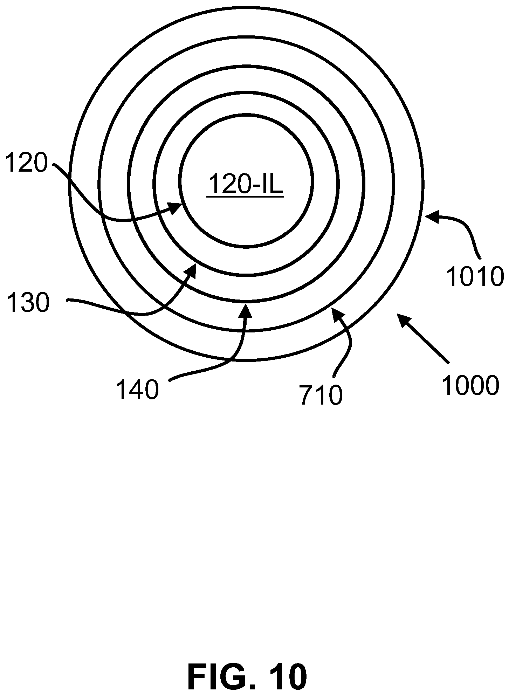

Steps 1 to 6 could also be defined, in another embodiment, as the steps of creating a tube with an inner diameter and final length of the intended graft design, that then can be shaped into a ring and mounted on a second target, where it will roll up a thin electrospun layer, thus forming a rolled layered graft around that tube.
7. A layer of a polymer **710** is electrospun over the collector **610** (not over ring-shaped structure **620).**
8. With respect to **FIG. 7****,** ring-shaped structure **620** is rolled from left to right over collector **610** and while rolling ring-shaped structure **620** is thickened as it rolls polymer **710** up over the outside surface of ring-shaped structure **620.** In other words, ring-shaped structure **620** is thickened by polymer **710** as it rolls from left to right resulting in a thicker ring-shaped structure **810 (****FIG. 8****).** Needless to say, but due to the rolling and adding polymer **710,** the diameter of ring-shaped structure **810** is bigger than the diameter of ring-shaped structure **620.** Rolled ring-shaped structure is now referred to as ring-shaped structure **810.** In one embodiment, one could consider rolling right after the electrospinning step, i.e. while the fibers are still a bit wet. This could increase the adhesion between the rolled layers, which may be better for structural integrity.
9. Ring-shaped structure **810** is now cut, removed from collector **610** and rigid pin **630,** that was used to shape it around collector **610,** removed resulting in cut ring-shaped structure **900 (****FIG. 9****).**
10. A rod (e.g. a stainless-steel rod) is placed in the inner lumen of cut ring-shaped structure straightening the structure (not shown).
11. Now in a straight configuration a polymer **1010** is electrospun over the outer surface of structure resulting in structure **1000 (****FIG. 10****).**
12. The next step is to remove the silicon tube (SI) **120** from structure **1000** which is accomplished by dissolving Polyethylene Oxide (PEO) layer **130** from structure **1000.** Submerging structure **1000** in water would allow one to remove silicon tube (SI) **120** from structure **1000** resulting in structure **1100.** The layer over layer structure of the three polymers layers allow for sliding of these layers over each other which prevents kinking of the tubular graft implant. Note that the middle polymer layer has a spiral rolled configuration, while the inner and outer layer have a continuous cylindrical configuration
13. Anneal structure **1100** in a straight configuration in a vacuum oven at about 37 degrees Celsius. The goal here is to end up with a straight cylindrical tubular graft implant.

Key to the invention is the steps of rolling over a thin layer of an electrospun polymer sheet to collect the sheet around the ring, thus creating a layered graft with better kink resistance. The steps of creating the ring structure are one example of achieving this, but there are other approaches to ensure the removal of a target and the invention is not limited to the specific example provided herein.

The essential requirements of the ring are:
- Defines the inner diameter and final length of final graft device.
- Needs to be flexible enough to be able to form a ring.
- Needs to be sufficiently sticky to the electrospun polymer layer to be able to collect the polymer when rolling over it.
- Needs to be easily removable from the inside of the rolled-up tube.

In yet another embodiment one can imagine, having a continuous inner tube (created in step 3) and a continuous outer tube (created in step 11) as these prevent the roll from unwinding.

The method and ultimate structure of the method have the following advantages.

### Burst pressure

A rolled tube was produced according to steps 1 to 13 and compared to another tube of the same materials and thickness that was produced by conventional electrospinning, i.e. direct deposition of polymer fibers on a rotating cylindrical target. Burst pressure testing showed burst pressures of 2000 and 800mmHg, respectively. In other words, the tubular graft implant has a significantly higher burst pressure resistance (around 2000 mmHg) compared to a regular spun graft (around 800 mmHg).

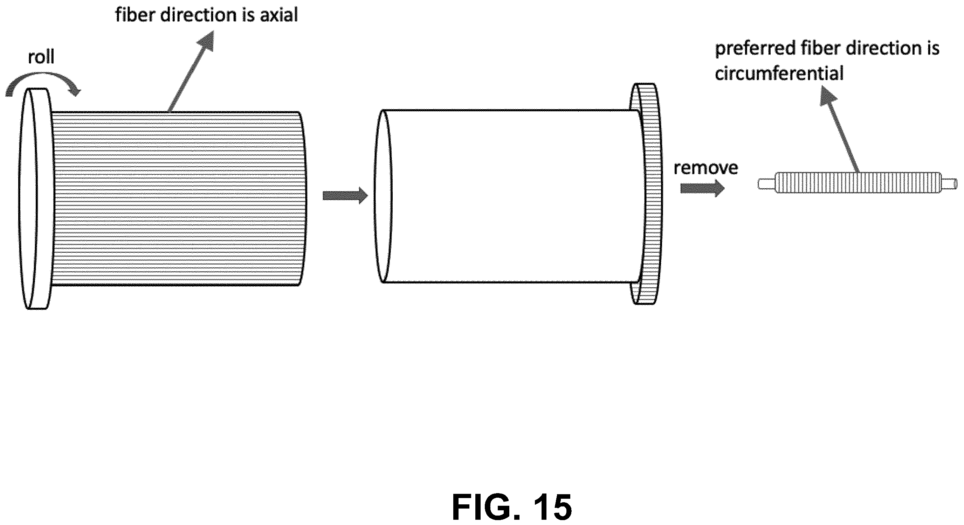

By producing a tube in the described way, the fiber orientation in the scaffold is turned by 90° when considering the circumferential direction **(****FIG. 15****).** As such the fiber alignment gets inverted from strong longitudinal alignment (i.e. polymer is stronger axially than it is circumferentially) to strong circumferential alignment of the fibers. The conventional way of spinning polymer on a cylindrical target typically results in a mild axial alignment (e.g. about 2:1 axial to circumferential tension module ratio). After rolling-up the main direction of the fibers in the described method the strong alignment ratio is turned into the circumferential direction. This will improve the kinking properties of the tube and the burst pressure resistance, radial force of the scaffold.

### Multiple layers

The tubular graft implant has multiple layers. Due to the layout with different layers instead of one thick scaffold layer the tubular graft implant now has crushing zones which are created between the multiple layers. These crushing zones allow squeezing of the material. When the tubular graft implant is bent the different layers could slide or move relative to each other thereby reducing the bending stiffness of the scaffold, and facilitating bending with superior kink resistance compared to the standard way of electrospinning a vascular graft.

Referring to crushing zones, the main point is that the layers can move relative to each other, and therefore they behave as a bundle of semi-independent thin structures. This makes the bending stiffness and therefore bendability of the composite structure much better. The main goal to obtain these so-called crushing zones is that the layers are not connected to each other.

In one embodiment, a method of the invention is a graft where the layers have different fiber orientations. This is obtained if one would have to spin two different sheets, then put them on top of each other (with different fiber orientation relative to one another) and then roll the two sheets together to form a graft/tube. Using only one sheet one would still get a multilayer graft, but all layers would have the same orientation. Accordingly, in one embodiment, the method includes the step of achieving different fiber orientations by rolling the layers.

### Porosity

The tubular graft implant has an acceptable/desirable porosity for ETR, this in contrast to e.g. ePTFE tubular grafts, which have a pore size that is insufficient to allow cell infiltration and ETR.

### Uniform thickness profile

The wall thickness profile of the tubular graft implants according to the method described herein will be more uniform than the profile achieved via standard electro-spinning due to elimination of potential thickness variations at the tips of the spun scaffold. This will allow a relatively long graft length for small diameter vessels (i.e. coronary bypass grafts).

In conventional electrospinning process the electrospun device typically ends up a bit thicker in the middle compared to the outer edges. This effect would be eliminated when the material is rolled because thickness is symmetric along the circumference of a spun tube. For the overall device one could have a thickness range of 0.1 mm to 2 mm, more typically 0.4 mm to 1 mm. One could have a diameter range of 2-8 mm, where a small diameter is typically 6 mm or less, however up to 8mm could be relevant for certain applications. The length of the device is between 5 cm to 100 cm.

For individual layers of the device, in one embodiment 30 micrometers to 50 micrometers is a preferred thickness. In another embodiment the thickness could be 20 micrometers to 100 micrometers. Noted is that the thickness is preferred to be thinner as that would be better for kink resistance, yet not too thin as that would be difficult to make uniform and reproduce.

### Metal-free solution

The tubular graft implants according to the method described herein do not have a support element (like metal), something which is common in the art to prevent kinking. The incorporation of a support element for the benefit of kink resistance and radial support is very expensive to manufacture in large amounts and entails significant quality and regulatory burden and also a risk of fracturing/failing long term in the clinic.

The tubular graft implants according to the method described herein focus on increasing the circumferential strength significantly and reducing bending stiffness to reach acceptable kink resistance. Therewith, a support element for such tubular graft implants is redundant. Another important design goal is to have a tubular graft implant that is fully bioabsorbable, which is achieved by the ultimate structure **1100.**

### Kinking

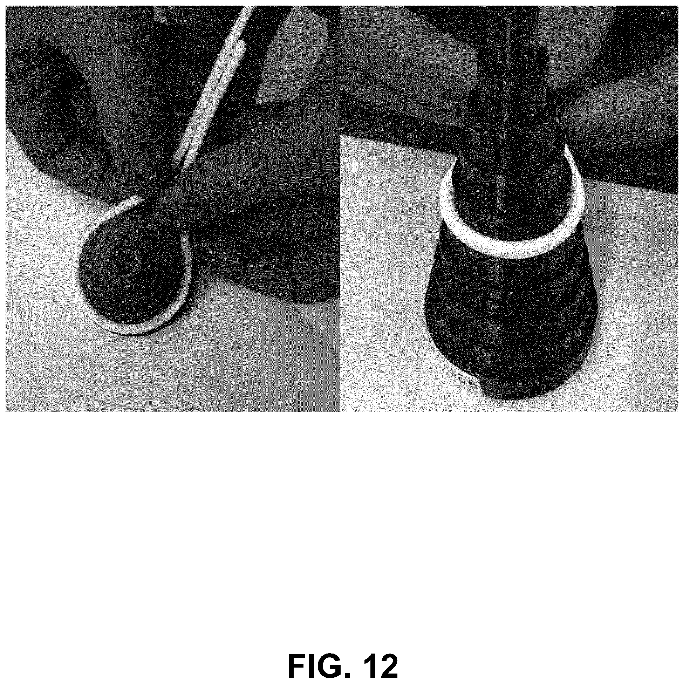

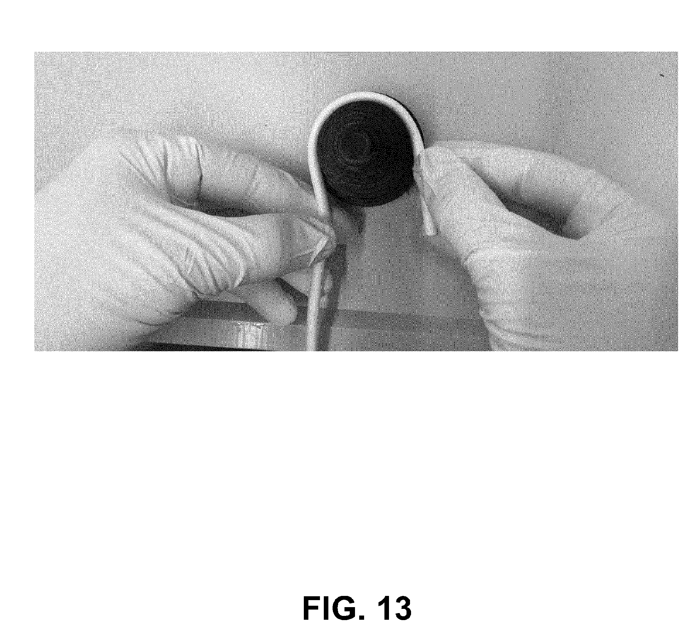

The tubular graft implants according to the method described herein were tested for kink resistance and have shown a substantially improved kink resistance compared to unsupported grafts. A prototype with ~600 µm in wall thickness and a diameter of ~3.2 mm is used as shown in **FIG. 12****.** Since rolled PEO layers were used as the inner diameter mandrel, the desirable 4.0mm inner diameter was not achieved. This prototype kinked at 1.25 cm radius while an unsupported graft kinks already before 3 cm radius. **FIG. 13** shows a prototype with a defined diameter of 4.0mm dictated by a fixed silicone tube. The wall thickness is ~500 µm. This prototype kinked at 1.75 cm, which is still a substantial increase compared to a standard electrospun graft (without metal support) kinking before 3 cm already. With respect to **FIG. 13****,** the bending radius for this prototype was 1.75 cm. A comparable single layer device had a kink radius of 3 cm. This difference is significant and a very relevant difference for applications intended for embodiments of this invention. The physical explanation for that significant difference is that bending stiffness of a material increases cubically when thickness increases. With multiple layers that can move relative to one another, they to a certain extent behave like several thin beams, rather than one thick beam. This results in a much lower bending force and therefore a more bendable kink-resistant device.

### Upscaling

Embodiments of the invention may also provide a benefit in allowing easier upscaling. For example, in an alternative embodiment one could consider that the electrospun sheet (step 7) for rolling up is produced separately using a much larger target, and cut to size and fitted on the target just prior to rolling (step 8).

As a further alternative embodiment, one could envision that the rolling is done on a straight target. For example,
- Step 1: Produce an inner layer by spinning on a straight cylindrical target with desired inner diameter.
- Step 2: Separately produce a thin electrospun sheet that will form the rolled inner spiral layer.
- Step 3: Roll the sheet produced in step 2 around the target with the inner layer produced in step 1.
- Step 4: Spin a thin outer layer around the rolled structure as produced in step 3.

Furthermore, one could envision an optional embodiment where the rolling is done in 2 parts. After the first rolling step, a reinforcement structure could be applied around the rolled structure, after which a second sheet would be rolled around that, followed by spinning of the outer layer. This would allow a process that is easy to scale and automate.

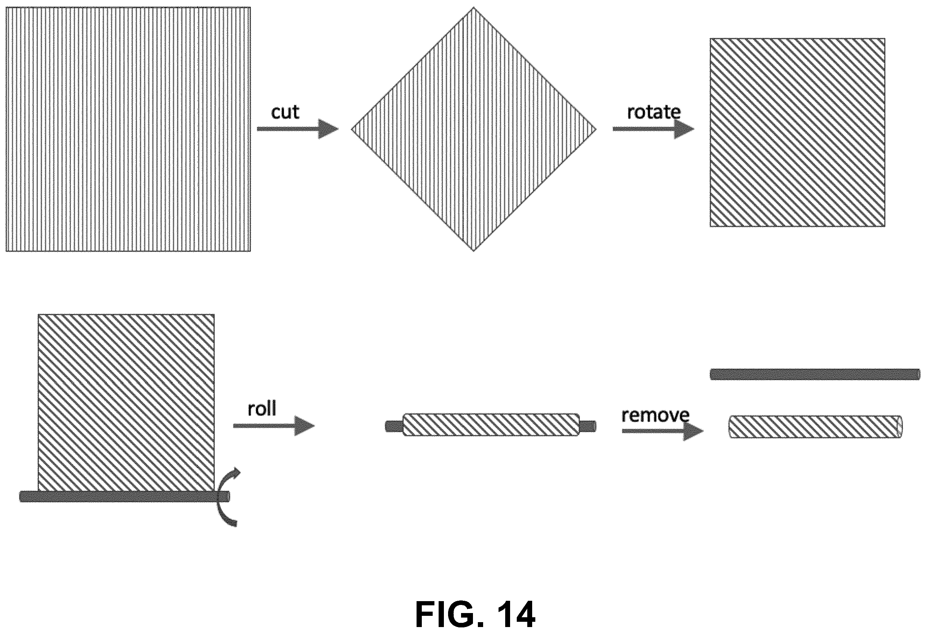

Furthermore, one could envision an optional embodiment where the rolling is done with a sheet that is cut in an angle to the preferred fiber orientation **(****FIG. 14****).** After cutting the sheet is rolled onto a mandrel resulting in a tube with a preferred fiber orientation at any desired angle relative to the main axis. In yet another embodiment, one could envision stacking multiple sheets at different preferred angles prior to rolling them into a mandrel. This way a graft could be created with multiple preferred fiber orientations, e.g. similar to the collagen fiber orientation in a native blood vessel, which has 2 symmetric preferred fiber orientations relative to the longitudinal axis.

With respect to **FIG. 14****:**

| | |
|---|---|
| Step 1: | Produce electrospun sheet with preferred fiber orientation (marked with lines). |
| Step 2: | Cut into a rectangular sheet with a different preferred fiber orientation. |
| Step 3: | Rotate sheet to align with rolling axis. |
| Step 4: | Roll sheet over cylindrical mandrel to produce multi-layer graft with preferred fiber orientation at angle with main graft axis. |
| Step 5: | Remove inner mandrel. |

For the purposes of this invention, the term graft is defined as grafts that are used to create a connection between two blood vessels, which could be a bypass graft, a shunt, an interposition graft, end-to-end, side-to-end, end-to-side, side-to-side, including snake and jump grafts (where several bypasses are made with one graft). What is not meant is devices that are used inside an existing blood vessel such as stents, endografts etc.. Small diameter ranges of graft devices provided herein are defined as 4 mm or less (for CABG), around 6 mm (for access graft) and up to 8 mm for peripheral grafts.

In one embodiment the tubular graft implant is a pre-bend graft. In the example that rolls a flexible thin tube over a large diameter mandrel results in a curved graft with a curve radius similar to the radius of the large mandrel. Accordingly, the graft would be pre-bent or precurved, which could have an advantage in some applications.

The electrospun material referenced in this document may comprise the ureido-pyrimidinone (UPy) quadruple hydrogen-bonding motif (pioneered by Sijbesma (1997), Science 278, 1601-1604) and a polymer backbone, for example selected from the group of biodegradable polyesters, polyurethanes, polycarbonates, poly(orthoesters), polyphosphoesters, polyanhydrides, polyphosphazenes, polyhydroxyalkanoates, polyvinylalcohol, polypropylenefumarate. Examples of polyesters are polycaprolactone, poly(L-lactide), poly(DL-lactide), poly(valerolactone), polyglycolide, polydioxanone, and their copolyesters. Examples of polycarbonates are poly(trimethylenecarbonate), poly(dimethyltrimethylenecarbonate), poly(hexamethylene carbonate).

The same result may be obtained with alternative, non-supramolecular polymers, if properties are carefully selected and material processed to ensure required surface characteristics. These polymers may comprise biodegradable or non-biodegradable polyesters, polyurethanes, polycarbonates, poly(orthoesters), polyphosphoesters, polyanhydrides, polyphosphazenes, polyhydroxyalkanoates, polyvinylalcohol, polypropylenefumarate. Examples of polyesters are polycaprolactone, poly(L-lactide), poly(DL-lactide), poly(valerolactone), polyglycolide, polydioxanone, and their copolyesters. Examples of polycarbonates are poly(trimethylenecarbonate), poly(dimethyltrimethylenecarbonate), poly(hexamethylene carbonate).

## Claims

1. A tubular electrospun graft implant, comprising:
(a) an electrospun tubular layer (140), wherein the electrospun tubular layer (140) has a longitudinal axis and an outer surface;
(b) one or more polymer layers (710) of an electrospun sheet rolled over the outer surface of the electrospun tubular layer (140) and around the longitudinal axis of the electrospun tubular layer (140) to provide a continuous inner lumen with a spiral rolled configuration; and
(c) a polymer layer (1010) electrospun over the rolled one or more polymer layers (710) of the electrospun sheet.

2. The tubular electrospun graft implant as set forth in claim 1, wherein there are between 10 to 20 layers.

3. The tubular electrospun graft implant as set forth in claim 1, wherein each of the one or more layers has a thickness between 30 micrometers to 50 micrometers or a thickness between 20 micrometers to 100 micrometers.

4. The tubular electrospun graft implant as set forth in claim 1, wherein there are two or more layers wherein the layers have different fiber directions relative to each other.

## Patentansprüche

1. Rohrformiges elektrogesponnenes Graft-Implantat, umfassend:
(a) eine elektrogesponnene rohrförmige Schicht (140), wobei die elektrogesponnene rohrförmige Schicht (140) eine Längsachse und eine Außenfläche aufweist;
(b) eine oder mehrere Polymerschichten (710) einer elektrogesponnenen Bahn, die über die Außenfläche der elektrogesponnenen rohrförmigen Schicht (140) und um die Längsachse der elektrogesponnenen rohrförmigen Schicht (140) gerollt sind, um ein kontinuierliches inneres Lumen mit einer spiralig aufgerollten Konfiguration bereitzustellen; und
(c) eine Polymerschicht (1010), die über die aufgerollte eine oder die mehreren Polymerschichten (710) der elektrogesponnenen Bahn elektrogesponnen ist.

2. Rohrformiges elektrogesponnenes Graft-Implantat nach Anspruch 1, wobei zwischen 10 bis 20 Schichten vorhanden sind.

3. Rohrformiges elektrogesponnenes Graft-Implantat nach Anspruch 1, wobei jede der einen oder mehreren Schichten eine Dicke zwischen 30 Mikrometer bis 50 Mikrometer oder eine Dicke zwischen 20 Mikrometer bis 100 Mikrometer aufweist.

4. Rohrformiges elektrogesponnenes Graft-Implantat nach Anspruch 1, wobei zwei oder mehr Schichten vorhanden sind, wobei die Schichten unterschiedliche Faserrichtungen relativ zueinander aufweisen.

## Revendications

1. Implant de greffe tubulaire électrofilé, comprenant :
(a) une couche tubulaire électrofilée (140), dans lequel la couche tubulaire électrofilée (140) possède un axe longitudinal et une surface extérieure ;
(b) une ou plusieurs couches de polymère (710) d'une feuille électrofilée enroulées sur la surface extérieure de la couche tubulaire électrofilée (140) et autour de l'axe longitudinal de la couche tubulaire électrofilée (140) pour fournir un lumen intérieur continu avec une configuration enroulée en spirale ; et
(c) une couche de polymère (1010) électrofilée sur les une ou plusieurs couches de polymère enroulées (710) de la feuille électrofilée.

2. Implant de greffe tubulaire électrofilé tel que décrit dans la revendication 1, dans lequel il comporte entre 10 et 20 couches.

3. Implant de greffe tubulaire électrofilé tel que décrit dans la revendication 1, dans lequel chacune des une ou plusieurs couches a une épaisseur comprise entre 30 micromètres et 50 micromètres ou une épaisseur comprise entre 20 micromètres et 100 micromètres.

4. Implant de greffe tubulaire électrofilé selon la revendication 1, dans lequel il existe deux ou plusieurs couches dans lequel les couches ont des directions de fibres différentes les unes par rapport aux autres.
